# EUROPEAN PATENT SPECIFICATION

(11) **EP 3 493 796 B1**
(45) Date of publication and mention of the grant of the patent: **15.04.2026**
(21) Application number: 17740032.2
(22) Date of filing: 21.07.2017
(51) Int. Cl.: A61K 31/198, A61P 13/12

(54) **GLUTAMINE FOR PRESERVING, IMPROVING OR RESTORING KIDNEY FUNCTION**
GLUTAMIN ZUR KONSERVIERUNG, VERBESSERUNG ODER WIEDERHERSTELLUNG DER NIERENFUNKTION
GLUTAMINE POUR LA PRÉSERVATION, L'AMÉLIORATION OU LA RESTAURATION DE LA FONCTION RÉNALE.

(30) Priority: 05.08.2016 EP 16182943
(43) Date of publication of application: 12.06.2019
(73) Proprietor: Fresenius Kabi Deutschland GmbH, 61352 Bad Homburg (DE)
(72) Inventor: WESTPHAL, Martin, 61350 Bad Homburg (DE); STOVER, John, 8006 Zürich (CH); BOTHE, Melanie, 60435 Frankfurt (DE); BAASNER, Silke, 61137 Schöneck (DE); ABELE, Rosa, 61462 Königstein (DE)
(74) Representative: Fresenius Kabi Deutschland GmbH
(86) International application number: PCT/EP2017/068448
(87) International publication number: WO 2018/024504

(56) References cited:
- WO-A1-2011/037970
- CLAUDIA MORRIS ET AL: "Metabolic Fate of Oral Glutamine Supplementation within Plasma and Erythrocytes of Patients with Sickle Cell Disease: Preliminary Pharmacokinetics Results | Blood | American Society of Hematology", BLOOD, vol. 116, no. 21, 19 November 2010 (2010-11-19), pages 1636, XP055670475
- NIKKI BUIJS ET AL: "Intravenous glutamine supplementation enhances renal de novo arginine synthesis in humans: a stable isotope study", THE AMERICAN JOURNAL OF CLINICAL NUTRITION, vol. 100, no. 5, 1 October 2014 (2014-10-01), US, pages 1385 - 1391, XP055670476, ISSN: 0002-9165, DOI: 10.3945/ajcn.113.081547
- CHRISTOPH FRAUNE ET AL: "AT 1 antagonism and renin inhibition in mice: pivotal role of targeting angiotensin II in chronic kidney disease", AMERICAN JOURNAL OF PHYSIOLOGY: RENAL PHYSIOLOGY, vol. 303, no. 7, 1 October 2012 (2012-10-01), United States, pages F1037 - F1048, XP055670482, ISSN: 1931-857X, DOI: 10.1152/ajprenal.00672.2011
- HSU CHI-YUAN: "Yes, AKI Truly Leads to CKD", JOURNAL OF THE AMERICAN SOCIETY OF NEPHROLOGY, vol. 23, no. 6, 1 June 2012 (2012-06-01), US, pages 967 - 969, XP055942950, ISSN: 1046-6673, Retrieved from the Internet <URL:http://dx.doi.org/10.1681/ASN.2012030222> DOI: 10.1681/ASN.2012030222
- LAKHMIR S. CHAWLA ET AL: "Acute kidney injury and chronic kidney disease: an integrated clinical syndrome", KIDNEY INTERNATIONAL, vol. 82, no. 5, 6 May 2012 (2012-05-06), GB, pages 516 - 524, XP055300221, ISSN: 0085-2538, DOI: 10.1038/ki.2012.208
- EMANUELA ESPOSITO ET AL: "Glutamine contributes to ameliorate inflammation after renal ischemia/reperfusion injury in rats", NAUNYN-SCHMIEDEBERG'S ARCHIVES OF PHARMACOLOGY, vol. 383, no. 5, 11 March 2011 (2011-03-11), DE, pages 493 - 508, XP055333292, ISSN: 0028-1298, DOI: 10.1007/s00210-011-0610-5
- HYUN-JUNG KIM ET AL: "Glutamine protects against cisplatin-induced nephrotoxicity by decreasing cisplatin accumulation", JOURNAL OF PHARMACOLOGICAL SCIENCES, vol. 127, no. 1, 1 January 2015 (2015-01-01), JP, pages 117 - 126, XP055333275, ISSN: 1347-8613, DOI: 10.1016/j.jphs.2014.11.009
- J.K. HWANG ET AL: "The Early Protective Effect of Glutamine Pretreatment and Ischemia Preconditioning in Renal Ischemia-Reperfusion Injury of Rat", TRANSPLANTATION PROCEEDINGS, vol. 45, no. 9, 1 November 2013 (2013-11-01), ORLANDO, FL; US, pages 3203 - 3208, XP055333280, ISSN: 0041-1345, DOI: 10.1016/j.transproceed.2013.08.028
- HIPPOCRATES YATZIDIS: "Oral supplement of six selective amino acids arrest progression renal failure in uremic patients", INTERNATIONAL UROLOGY AND NEPHROLOGY, KLUWER ACADEMIC PUBLISHERS, DO, vol. 36, no. 4, 1 December 2004 (2004-12-01), pages 591 - 598, XP019269710, ISSN: 1573-2584
- D. K. HEYLAND ET AL: "Glutamine and Antioxidants in the Critically Ill Patient: A Post Hoc Analysis of a Large-Scale Randomized Trial", JPEN - JOURNAL OF PARENTERAL AND ENTERAL NUTRITION, vol. 39, no. 4, 1 May 2015 (2015-05-01), US, pages 401 - 409, XP055333267, ISSN: 0148-6071, DOI: 10.1177/0148607114529994
- TATIANA CAROLINA ALBA-LOUREIRO ET AL: "Effects of glutamine supplementation on kidney of diabetic rat", AMINO ACIDS ; THE FORUM FOR AMINO ACID AND PROTEIN RESEARCH, SPRINGER-VERLAG, VI, vol. 38, no. 4, 17 June 2009 (2009-06-17), pages 1021 - 1030, XP019805400, ISSN: 1438-2199

## Description

### FIELD OF THE INVENTION

The present invention relates to L-glutamine for improving kidney function.

### BACKGROUND OF THE INVENTION

L-glutamine is one of the 20 naturally occurring amino acids in dietary protein. The levels required are elevated during periods of disease and muscle wasting typical of physical trauma.

L-glutamine is sold as an isolated amino acid. It is also found in high levels in dietary meats and eggs. L-glutamine has been shown to improve immunity, to reduce inflammation, to regulate cellular hydration and to improve gut barrier function, specifically in metabolically stressed patients, e.g. in critically ill patients, surgical patients and bone marrow transplant patients.

WO2011/037 A1 describes the treatment of proteinuria by modulation of intra-podocyte pH.

Esposito et al. 2011 (Naunyn-Schmied Arch Pharmacol (2011) 383: 493-508) describe beneficial effects of the administration of glutamine in connection with ischemia-reperfusion related inflammation.

Kim et al. 2015 (Journal of Pharmacological Sciences (2015) 127: 117-126) describe protective effects of glutamine against cisplatin-induced nephrotoxicity.

Hwang et al. 2013 (Transplantation Proceedings (2013) 45: 3203-3208) describe protective effects of glutamine in renal ischemia-reperfusion injury.

Alba-Loureiro 2009 (Amino Acids (2010) 38: 1021-1030) describes that short-term treatment with glutamine in association with increased glucose levels could cause alterations in glomerular morphology.

Fraune et al. (Am J Physiol Renal Physiol (2012) 303: 1037-1048) describe nephroprotective effects mediated by ACE and AT₁ receptor inhibition mediate nephroprotective effects.

Hsu 2012, in an editorial article (J Am Soc Nephrol (2012) 23: 967-969), opines that acute kidney injury leads to chronic kidney disease.

Chawla and Kimmel 2012 (Kidney International (2012) 82: 516-524) describe that acute kidney injury can cause end-stage renal disease and increase the risk of developing incident chronic kidney disease.

Yatzidis 2004 (International Urology and Nephrology (2004) 36: 591-598) describes that an oral supplement comprising 6 amino acids arrested progression of renal failure in uremic patients.

Morris et al. 2010 (Hemoglobinopathies, excluding Thalassemia: Poster I, November 19, 20210)) report that the oral supplementation of 10 g glutamine increased plasma and erythrocyte glutamine concentration in 3 patients with sickle cell disease.

Buijus et al. (Am J Clin Nutr (2014) 100: 1385-1391) describe that an intravenous glutamine supplement doubles renal arginine production from citrulline.

Due to the fact that orally administered L-glutamine is metabolized to citrulline, L-alanine and other amino acids by the intestinal epithelial cells (Souba WW, Smith RJ, Wilmore DW. Glutamine metabolism by the intestinal tract. JPEN J Parenter Enteral Nutr. 1985 Sep-Oct;9(5):608-17.), only intravenous administration of L-glutamine allows for direct and predictable manipulation of plasma L-glutamine levels. Thus, advantageously, glutamine is provided in form of compositions for parenteral administration.

However, L-glutamine is poorly soluble in aqueous solvents.

Furthermore, due to the heat instability of L-glutamine, compositions comprising glutamine may not be terminally sterilized by autoclaving.

Therefore, and in order to increase chemical stability, solubility and shelf life, L-glutamine is mainly provided in the form of dipeptides. L-glutamine is released from the peptide by hydrolysis.

Commercially available compositions for parenteral use comprising L-glutamine in form of dipeptides are sold under the brand names Dipeptiven^{™} (concentrate providing L-glutamine in form of L-alanyl-L-glutamine) and Glamin^{™} (a balanced amino acid solution providing L-glutamine in form of glycyl-L-glutamine) respectively. The products are used in parenteral nutrition, specifically in patients with moderate to severe catabolic status.

It has long been known that patients with renal insufficiency should not receive nutrition too high in protein in order to prevent hyperuremia.

In a large clinical trial (Heyland DK, Elke G, Cook D, Berger MM, Wischmeyer PE, Albert M, Muscedere J, Jones G, Day AG; Canadian Critical Care Trials Group: Glutamine and Antioxidants in the critically ill patient: A post hoc analysis of a large-scale randomized trial. JPEN J Parenter Enteral Nutr. 2015 May;39(4):401-9. doi: 0.1177/0148607114529994. Epub 2014 May 5.) it has recently been shown that acute renal failure is a contraindication for parenteral glutamine administration.

The use of Dipeptiven^{™} and Glamin^{™} respectively is contraindicated in patients with severe renal insufficiency, i.e. in patients with a creatinine clearance below 25 ml/minute. This is explicitly mentioned in the summary of product characteristics (SmPC) of both products. It would thus have been expected that L-glutamine may be harmful with respect to kidney function, particularly in patients with renal insufficiency.

### SUMMARY OF THE INVENTION

The present inventors have surprisingly found that providing L-glutamine parenterally not only does not impair but even supports kidney function.

### DETAILED DESCRIPTION OF THE INVENTION

The present disclosure relates to L-glutamine for use in preserving, improving or restoring kidney function or for decelerating and/or attenuating a decline in kidney function, wherein improving or restoring kidney function or decelerating and/or attenuating a decline in kidney function involves the treatment of chronic renal disease, preferably chronic progressive nephropathy, and wherein improving or restoring kidney function or decelerating and/or attenuating a decline in kidney function involves the treatment or prevention of glomerulosclerosis and/or tubular basophilia and/or tubular atrophy and/or thickening of the tubular basement membrane. L-glutamine is administered parenterally, preferably intravenously.

In the context of the present disclosure L-glutamine is provided in a composition which is administered parenterally. Preferably it is administered in the form of an aqueous solution. The composition may be a ready-to-use injectable liquid, a concentrate requiring dilution before administration or a powder for the preparation of a liquid composition for parenteral administration.

The composition providing L-glutamine may comprise L-glutamine as a free amino acid, as a dipeptide, as a tripeptide or as a tetrapeptide. In the context of the present disclosure, the composition preferably provides L-glutamine in form of a dipeptide, e.g. L-alanyl-L-glutamine or glycyl-L-glutamine. Most preferably, the composition provides L-glutamine in form of L-alanyl-L-glutamine.

When L-glutamine is provided in form of L-alanyl-L-glutamine, the composition suitably comprises 5 to 550 mg, preferably 10 to 400 mg, more preferably 20 to 250 mg L-alanyl-L-glutamine per ml.

When L-glutamine is provided in form of glycyl-L-glutamine, the composition suitably comprises 1 to 300 mg, preferably 5 to 200 mg, more preferably 10 to 100 mg glycyl-L-glutamine per ml.

In the context of the present disclosure the composition providing L-glutamine may provide L-glutamine or a source of L-glutamine, i.e. L-glutamine in form of di-, tri or tetrapeptide, as the only active ingredient.

However, it may also comprise further active ingredients.

It may for example comprise further amino acids, e.g. it may be a balanced amino acid solution.

The composition providing L-glutamine may further comprise pharmaceutically acceptable excipients, e.g. tonicity agents, agents for pH adjustment, antioxidants or antimicrobial agents.

### The antioxidant

The composition may comprise at least one pharmaceutically acceptable antioxidant.

An antioxidant useful in the composition in the context of the disclosure may be any pharmaceutically acceptable compound having antioxidant activity, for example, the antioxidant may be selected form the group consisting of sodium metasulfite, sodium bisulfite, sodium sulfite, sodium thiosulfate, thioglycerol, thiosorbitol, thioglycolic acid, cysteine hydrochloride, n-acetly-cysteine, citric acid, alpha-tocopherol, beta-tocopherol, gamma-tocopherol, soluble forms of vitamin E, butylated hydroxyanisole (BHA), butylated hydroxytoluene (BHT), t-butylhydroquinone (TBHQ), monothioglycerol, propyl gallate, histidine, enzymes such as superoxide dismutase, catalase, selenium glutathione peroxidase, phospholipid hydroperoxide and glutathione peroxidase, Coenzyme Q10, tocotrienols, carotenoids, quinones, bioflavonoids, polyphenols, bilirubin, ascorbic acid, isoascorbic acid, uric acid, metal-binding proteins, ascorbic acid palmitate, an antioxidant obtained or obtainable from rosemary, rosemary extract and mixtures thereof.

If present, the total amount of agents with antioxidant activity is preferably in the range of from 0.001 wt.% to 0.05 wt. %, more preferably from 0.01 wt.% to 0.04 wt.%, more preferably from 0.01 wt.% to 0.03 wt.%, and even more preferably from 0.015 wt.% to 0.025 wt.% based on the total weight of the composition.

### The tonicity agent

The composition in the context of the present disclosure may comprise at least one pharmaceutically acceptable tonicity agent.

Tonicity agents are used to confer tonicity. Suitable tonicity agents may be selected from the group consisting of sodium chloride, mannitol, lactose, dextrose, sorbitol and glycerol.

Preferably, the total amount of tonicity agents is in the range of 0.1 to 10 wt.%, more preferably from 1 wt.% to 5 wt.%, more preferably from 1 wt.% to 4 wt.%, more preferably 1 wt.% to 3 wt.%, more preferably from 1.5 wt.% to 2.8 wt.%, and even more preferably from 2.0 wt.% to 2.8 wt.% based on the total weight of the emulsion.

Preferably, the composition as parenterally administered has an osmolality in the range of from 200 to 900, more preferably of from 250 to 600, most preferably of from 300 to 450 mOsmol/kg, measured with a Vapor Pressure Osmometer, Model 5520 (Vapro TM) according to USP <785>.

### pH adjustment

The pH of the composition may be adjusted by adding solutions of conventionally known acids or bases such as HCl and NaOH or through the use of buffers, such as phosphate or citrate buffers.

The final pH of the composition is preferably in the range of from 5.5 to 7.5.

### The antimicrobial agent

The composition may comprise at least one pharmaceutically acceptable antimicrobial agent.

An antimicrobial agent useful in the composition in the context of the present disclosure may be any pharmaceutically acceptable compound having antimicrobial activity, for example, the antimicrobial agent may be selected form the group consisting of benzalkonium chloride, benzethonium chloride, benzyl alcohol, chlorobutanol, chlorocresol, cresol, parabens (methyl, ethyl, propyl, butyl esters), phenol, phenylmercuric nitrate and thimerosol.

If present, the total amount of agents with antimicrobial activity is preferably in the range of from 0.001 wt.% to 2.5 wt. %, more preferably from 0.005 wt.% to 1.5 wt.%, more preferably from 0.01 wt.% to 1.1 wt.%, and even more preferably from 0.01 wt.% to 0.5 wt.% based on the total weight of the composition.

It is to be understood that the composition providing L-glutamine, in order to be suitable for parenteral administration, has to be sterile and pyrogen-free which is accomplished by aseptic manufacturing and filling techniques and/or by terminally sterilizing the compositions in an autoclave.

The composition may be manufactured according to standard methods known in the art, e.g. by dissolving the ingredients in a suitable solvent or solvent mixture. The resulting solution may optionally be lyophilized.

The composition providing L-glutamine is administered parenterally as a medicament.

However, the composition providing L-glutamine may also be mixed with other compositions, e.g. with lipid emulsions, amino acid solutions and carbohydrate solutions in the context of a parenteral nutrition regimen.

It may also be administered as a parenteral supplement to an enteral nutrition regimen.

The present disclosure includes inter alia the following aspects:
In a first aspect the present disclosure relates to L-glutamine for use in preserving, improving or restoring kidney function or for decelerating and/or attenuating a decline in kidney function, wherein L-glutamine is provided in a composition that is administered parenterally, preferably intravenously, wherein improving or restoring kidney function or decelerating and/or attenuating a decline in kidney function involves the treatment of chronic renal disease, preferably chronic progressive nephropathy, and wherein improving or restoring kidney function or decelerating and/or attenuating a decline in kidney function involves the treatment or prevention of glomerulosclerosis and/or tubular basophilia and/or tubular atrophy and/or thickening of the tubular basement membrane.

In a second aspect the present disclosure relates to L-glutamine for use according to aspect 1, wherein the treatment involves the deceleration and/or attenuation of the development and/or the reversal of glomerulosclerosis and/or tubular basophilia and/or tubular atrophy and/or thickening of the tubular basement membrane.

In a third aspect the present disclosure relates to L-glutamine for use according to aspect 1 or 2 in patients with renal insufficiency.

In a fourth aspect the present disclosure relates to L-glutamine for use according to aspect 3, wherein the renal insufficiency involves a reduced glomerular filtration rate (GFR).

In a fifth aspect the present disclosure relates to L-glutamine for use according to aspect 3 or 4, wherein the renal insufficiency involves a creatinine clearance of 15 to 89 ml per minute.

In an sixth aspect the present disclosure relates to L-glutamine for use according to any of aspects 3 to 5, wherein the renal insufficiency involves a creatinine clearance of 60 to 89 ml per minute.

In a seventh aspect the present disclosure relates to L-glutamine for use according to any of aspects 3 to 5, wherein the renal insufficiency involves a creatinine clearance of 45 to 59 ml per minute.

In a eighth aspect the present disclosure relates to L-glutamine for use according to any of aspects 3 to 5, wherein the renal insufficiency involves a creatinine clearance of 25 to 44 ml per minute.

In an ninth aspect the present disclosure relates to L-glutamine for use according to any of the preceding aspects in patients suffering from any of diabetes, ischemia, infection, intoxication, hypertension, sepsis, focal segmental glomerulosclerosis, reflux nephropathy, sickle cell disease, autoimmune disease, malnutrition, cachexia, inflammation, hyperuricemia, and/or in patients admitted to an intensive care unit and/or in trauma patients and/or in post-surgical patients.

In a tenth aspect the present disclosure relates to L-glutamine for use according to any of the preceding aspects, wherein the daily glutamine dose is 50 to 5000, preferably 100 to 4500 mg per kg bodyweight.

In a eleventh aspect the present disclosure relates to L-glutamine for use according to any of the preceding aspects, wherein the composition provides 5 to 500 mg, preferably 10 to 300 mg, more preferably 15 to 150 mg L-glutamine per ml.

In a twelfth aspect the present disclosure relates to L-glutamine for use according to any of the preceding aspects, wherein the composition comprises L-glutamine as a free amino acid or in form of a dipeptide, tripeptide or tetrapeptide, preferably in form of a dipeptide, more preferably in form of L-alanyl-L-glutamine or glycyl-L-glutamine.

In a thirteenth aspect the present disclosure relates to L-glutamine for use according to any of the preceding aspects, wherein the composition comprises 5 to 550 mg, preferably 10 to 400 mg, more preferably 20 to 250 mg L-alanyl-L-glutamine per ml.

In a fourteenth aspect the present disclosure relates to L-glutamine for use according to any of the aspects 1 to 14, wherein the composition comprises 1 to 300 mg, preferably 5 to 200 mg, more preferably 10 to 100 mg glycyl-L-glutamine per ml.

### Embodiments

L-glutamine for use in preserving, improving or restoring kidney function or for decelerating and/or attenuating a decline in kidney function, wherein L-glutamine is provided in a composition that is administered parenterally, preferably intravenously, wherein the preserving, improving or restoring kidney function or the decelerating and/or attenuating a decline in kidney function involves the treatment of chronic renal disease, preferably chronic progressive nephropathy, and wherein the preserving, improving or restoring kidney function or the decelerating and/or attenuating a decline in kidney function involves the treatment or prevention of glomerulosclerosis and/or tubular basophilia and/or tubular atrophy and/or thickening of the tubular basement membrane.

### EXAMPLES

The effect of L-glutamine (administered in form of L-alanyl-L-glutamine; Dipeptiven^{™}) following daily continuous intravenous infusion was examined in a 5/6 kidney nephrectomised rat model.

L-alanyl-L-glutamine was administered for 9 consecutive days. The effects attributable to L-glutamine (provided in form of alanyl-glutamine; Dipeptiven^{™}) were determined by comparison to an L-alanine infusion.

To prevent protein overdose, the protein content of the animal food was adapted according to the L-alanyl-L-glutamine or L-alanine dose administered intravenously.

### Experimental procedures

The study was conducted according to the following design:

| Group | Treatment | Dose level (mg/kg /day) | Dose (mL/ kg/ day) | volume (mL/ kg/ h) | Dose concentration (mg/mL) | Diet Protein % | Number of males |
|---|---|---|---|---|---|---|---|
| 2. Control 2 | Vehicle | 0 | 37.5 | 1.5625 | 0 | 12 | 10 |
| 3. 0.5 Ala-Gln | Dipeptiven^{™} | 500 | 37.5 | 1.5625 | 13.33 | 12 | 10 |
| 4. 3.0 Ala-Gln | Dipeptiven^{™} | 3000 | 37.5 | 1.5625 | 80 | 9 | 10 |
| 5. 7.5 Ala-Gln | Dipeptiven^{™} | 7500 | 37.5 | 1.5625 | 200 | 4.5 | 10 |
| 6. 7.5 Ala-Gln | Dipeptiven^{™} | 7500 | 37.5 | 1.5625 | 200 | 12 | 10 |
| 7. 1.2 Ala | Alanine | 1200 | 37.5 | 1.5625 | 32 | 10.8 | 10 |
| 8. 3.0 Ala | Alanine | 3000 | 37.5 | 1.5625 | 80 | 9 | 10 |

| | | | | | | | |
|---|---|---|---|---|---|---|---|
| Ala-Gln = L-alanyl-L-glutamine; N(2)-L-alanyl-L-glutamine Ala = L-alanine | | | | | | | |

Group 1 and 2 animals (control) received the vehicle (0.9 % NaCl).

Groups 5 and 6 received Dipeptiven^{™} without any dilution, i.e. L-alanyl-L-glutamine in a concentration of 200 mg/ml.

Group 3 and 4 animals received diluted Dipeptiven^{™}, i.e. L-alanyl-L-glutamine in a concentration of 13.33 and 80 mg/ml respectively.

Group 7 and 8 animals received L-alanine in a concentration of 32 and 80 mg/ml respectively.

All animals in groups 1 and 3 to 8 underwent a 5/6 nephrectomy. All animals in group 2 were "sham operated", meaning the abdomen was opened and closed in the surgery, but the kidneys were not excised and remained in the body.

During the acclimatisation period, a polyurethane catheter was implanted into the posterior vena cava via the left femoral vein. Following implantation, the animals were maintained on continuous infusion with physiological saline prior to the start of treatment.

Morbidity/mortality checks were performed at least twice daily. Clinical observations were performed daily. A full clinical examination was performed daily. Individual body weights were recorded three times a week. Food consumption was measured three times a week for each cage of animals, including one measurement during the acclimatisation period. Clinical laboratory determinations were performed on selected animals on days -1 and on days 1 to 10. Animals were sampled for amino acid determination on days 1, 4, 7 and 10. Animals were fasted before blood sampling performed on day 10.

All animals were killed at the end of the treatment period (day 10) and necropsied. Organ/tissue samples were fixed and preserved at necropsy for all animals. Selected organs/tissues from all animals were examined histopathologically. In addition, selected organs/tissues from all animals were used for amino acid determination.

### Results

No treatment-related clinical signs or changes in blood levels of biomarkers for kidney and liver injury were noted throughout the treatment period for animals treated with L-alanyl-L-glutamine and for those treated with L-alanine whatever the dose and diet.

The intravenous infusion of L-alanyl-L-glutamine and L-alanine respectively for 9 days in a 5/6 kidney nephrectomised rat model was overall well tolerated.

The infusion of L-alanyl-L-glutamine led to a decreased incidence of chronic progressive nephropathy.

In detail, the intravenous administration of L-alanyl-L-glutamine to nephrectomised rats by continuous infusion over a period of 9 days in doses of up to 7500 mg/kg/day or L-alanine in doses of up to 3000 mg/kg/day led to the following test items related changes in the incidence of chronic progressive nephropathy: Control: 3/5 animals, L-alanyl-L-glutamine 0.5 g/kg/day: 2/5 animals, L-alanyl-L-glutamine 3.0 g/kg/day: 0/5 animals, L-alanyl-L-glutamine 7.5 g/kg/day: 0/5 animals, L-alanyl-L-glutamine 7.5 g/kg/day (+ 12% protein in diet): 2/5 animals, L-alanine 1.2 g/kg/day: 4/5 animals, L-alanine 3.0 g/kg/day: 3/5 animals. These results are depicted in the table below:

| | Group 1 control | Group 2 sham | Group 3 0.5 g/kg Ala-Gln | Group 4 3.0 g/kg Ala-Gln | Group 5 7.5 g/kg Ala-Gln | Group 6 7.5 g/kg Ala-Gln | Group 7 1.2 g/kg Ala | Group 8 3.0 g/kg Ala |
|---|---|---|---|---|---|---|---|---|
| Protein in diet | 12% | 12% | 12% | 9% | 4.5% | 12% | 10.8% | 9% |
| Nephropathy | 3/5 | 0/5 | 2/5 | 0/5 | 0/5 | 1/5 | 4/5 | 3/5 |

Except for group 2 ("sham" operated) animals, all animals underwent 5/6 nephrectomy, meaning 5/6 of the kidney tissue was removed. During the treatment period, all animals received the recommended amount of protein for this age group, namely 12% of protein in the diet.

In the groups receiving L-alanyl-L-glutamine or L-alanine, extra amino acids were supplied intravenously in form of a L-alanyl-L-glutamine (Dipeptiven^{™}) or L-alanine respectively. To prevent an excess supply of amino acids i.e. an excess supply of nitrogen, the amount of protein in the diet was reduced accordingly.

In group 1 the animals underwent nephrectomy and received saline plus 12% protein in the diet. 60% (3/5) of these animals developed a chronic progressive nephropathy.

In group 2, the animals retained their kidneys and received saline plus 12% protein the diet. None of these animals developed a chronic progressive nephropathy.

All animals in groups 3 to 8 underwent nephrectomy and were treated with either L-alanyl-L-glutamine or L-alanine. L-alanyl-L-glutamine treatment (in groups 3, 4, and 5) reduced the occurrence of chronic progressive nephropathy dose-dependently from 60% to 40% to 0%, while L-alanine treatment did not.

Of note, animals in group 6 received both a high dose of glutamine and 12% protein in the diet, thus an amino acid overload. Amino acid/protein/nitrogen overload is known to increase the occurrence of chronic progressive nephropathy. Still only 20 % (1/5) of the animals in this group developed chronic progressive nephropathy.

The increasing doses of glutamine were adjusted for by decreasing the dosages of protein in the diet in groups 3, 4, and 5 respectively.

Low protein diet is known to reduce the occurrence of chronic progressive nephropathy and thus might be suggested as the cause of the beneficial effect.

This is, however, easily disproven by comparing groups 4 and 8. Both groups received a 9% protein diet. Group 4 animals were treated with L-alanyl-L-glutamine and no chronic nephropathy occurred, while group 8 animals were treated with L-alanine and 60% of the animals developed a chronic progressive nephropathy. Thus, the beneficial effect is not due to the decreased amount of protein in the diet, but due to the L-alanyl-L-glutamine treatment.

These experiments clearly demonstrate that parenteral administration of L-glutamine reduces the occurrence of chronic progressive nephropathy in a dose-dependent manner.

## Claims

1. L-glutamine for use in preserving, improving or restoring kidney function or for decelerating and/or attenuating a decline in kidney function, wherein L-glutamine is provided in a composition that is administered parenterally, preferably intravenously, wherein the preserving, improving or restoring kidney function or the decelerating and/or attenuating a decline in kidney function involves the treatment of chronic renal disease, preferably chronic progressive nephropathy, and wherein the preserving, improving or restoring kidney function or the decelerating and/or attenuating a decline in kidney function involves the treatment or prevention of glomerulosclerosis and/or tubular basophilia and/or tubular atrophy and/or thickening of the tubular basement membrane.

2. L-glutamine for use according to claim 1, wherein the treatment involves the deceleration and/or attenuation of the development and/or the reversal of glomerulosclerosis and/or tubular basophilia and/or tubular atrophy and/or thickening of the tubular basement membrane.

3. L-glutamine for use according to any of the preceding claims in patients with renal insufficiency.

4. L-glutamine for use according to claim 5, wherein the renal insufficiency involves a reduced glomerular filtration rate (GFR).

5. L-glutamine for use according to claim 5 or 6, wherein the renal insufficiency involves a creatinine clearance of 15 to 89 ml per minute.

6. L-glutamine for use according to any of claims 5 to 7, wherein the renal insufficiency involves a creatinine clearance of 60 to 89 ml per minute.

7. L-glutamine for use according to any of claims 5 to 7, wherein the renal insufficiency involves a creatinine clearance of 45 to 59 ml per minute.

8. L-glutamine for use according to any of claims 5 to 7, wherein the renal insufficiency involves a creatinine clearance of 25 to 44 ml per minute.

9. L-glutamine for use according to any of the preceding claims in patients suffering from any of diabetes, ischemia, infection, intoxication, hypertension, sepsis, focal segmental glomerulosclerosis, reflux nephropathy, sickle cell disease, autoimmune disease, malnutrition, cachexia, inflammation, hyperuricemia, and/or in patients admitted to an intensive care unit and/or in trauma patients and/or in post-surgical patients.

10. L-glutamine for use according to any of the preceding claims, wherein the daily glutamine dose is 50 to 5000, preferably 100 to 4500 mg per kg bodyweight.

11. L-glutamine for use according to any of the preceding claims, wherein the composition provides 1 to 500 mg, preferably 5 to 300 mg, more preferably 10 to 150 mg L-glutamine per ml.

12. L-glutamine for use according to any of the preceding claims, wherein the composition comprises L-glutamine as a free amino acid or in form of a dipeptide, tripeptide or tetrapeptide, preferably in form of a dipeptide, more preferably in form of L-alanyl-L-glutamine or glycyl-L-glutamine.

13. L-glutamine for use according to any of the preceding claims, wherein the composition comprises 5 to 550 mg, preferably 10 to 400 mg, more preferably 20 to 250 mg L-alanyl-L-glutamine per ml.

14. L-glutamine for use according to any of claims 1 to 14, wherein the composition comprises 1 to 300 mg, preferably 5 to 200 mg, more preferably 10 to 100 mg glycyl-L-glutamine per ml.

## Patentansprüche

1. L-Glutamin zur Verwendung bei der Konservierung, Verbesserung oder Wiederherstellung der Nierenfunktion oder zum Verlangsamen und/oder Abschwächen einer Abnahme der Nierenfunktion, wobei L-Glutamin in einer Zusammensetzung bereitgestellt wird, die parenteral, vorzugsweise intravenös, verabreicht wird, wobei die Konservierung, Verbesserung oder Wiederherstellung der Nierenfunktion oder das Verlangsamen und/oder Abschwächen einer Abnahme der Nierenfunktion die Behandlung einer chronischen Nierenerkrankung, vorzugsweise einer chronisch fortschreitenden Nephropathie beinhaltet, und wobei die Konservierung, Verbesserung oder Wiederherstellung der Nierenfunktion oder die Verlangsamung und/oder Abschwächung einer Abnahme der Nierenfunktion die Behandlung oder Vorbeugung von Glomerulosklerose und/oder tubulärer Basophilie und/oder tubulärer Atrophie und/oder Verdickung der tubulären Basalmembran beinhaltet.

2. L-Glutamin zur Verwendung nach Anspruch 1, wobei die Behandlung die Verlangsamung und/oder Abschwächung der Entwicklung und/oder die Umkehrung von Glomerulosklerose und/oder tubulärer Basophilie und/oder tubulärer Atrophie und/oder Verdickung der tubulären Basalmembran beinhaltet.

3. L-Glutamin zur Verwendung nach einem der vorhergehenden Ansprüche bei Patienten mit Niereninsuffizienz.

4. L-Glutamin zur Verwendung nach Anspruch 5, wobei die Niereninsuffizienz eine reduzierte glomeruläre Filtrationsrate (GFR) beinhaltet.

5. L-Glutamin zur Verwendung nach Anspruch 5 oder 6, wobei die Niereninsuffizienz mit einer Kreatinin-Clearance von 15 bis 89 ml pro Minute einhergeht.

6. L-Glutamin zur Verwendung nach einem der Ansprüche 5 bis 7, wobei die Niereninsuffizienz mit einer Kreatinin-Clearance von 60 bis 89 ml pro Minute einhergeht.

7. L-Glutamin zur Verwendung nach einem der Ansprüche 5 bis 7, wobei die Niereninsuffizienz mit einer Kreatinin-Clearance von 45 bis 59 ml pro Minute einhergeht.

8. L-Glutamin zur Verwendung nach einem der Ansprüche 5 bis 7, wobei die Niereninsuffizienz mit einer Kreatinin-Clearance von 25 bis 44 ml pro Minute einhergeht.

9. L-Glutamin zur Verwendung nach einem der vorhergehenden Ansprüche bei Patienten, die an Diabetes, Ischämie, Infektion, Vergiftung, Bluthochdruck, Sepsis, fokaler segmentaler Glomerulosklerose, Refluxnephropathie, Sichelzellenkrankheit, Autoimmunerkrankung, Unterernährung, Kachexie, Entzündung, Hyperurikämie leiden, und/oder bei Patienten, die auf einer Intensivstation behandelt werden, und/oder bei Traumapatienten und/oder bei Patienten nach einer Operation.

10. L-Glutamin zur Verwendung nach einem der vorhergehenden Ansprüche, wobei die tägliche Glutamindosis 50 bis 5000, vorzugsweise 100 bis 4500 mg pro kg Körpergewicht beträgt.

11. L-Glutamin zur Verwendung nach einem der vorhergehenden Ansprüche, wobei die Zusammensetzung 1 bis 500 mg, vorzugsweise 5 bis 300 mg, besonders bevorzugt 10 bis 150 mg L-Glutamin pro ml bereitstellt.

12. L-Glutamin zur Verwendung nach einem der vorhergehenden Ansprüche, wobei die Zusammensetzung L-Glutamin als freie Aminosäure oder in Form eines Dipeptids, Tripeptids oder Tetrapeptids umfasst, vorzugsweise in Form eines Dipeptids, noch bevorzugter in Form von L-Alanyl-L-Glutamin oder Glycyl-L-Glutamin.

13. L-Glutamin zur Verwendung nach einem der vorhergehenden Ansprüche, wobei die Zusammensetzung 5 bis 550 mg, vorzugsweise 10 bis 400 mg, besonders bevorzugt 20 bis 250 mg L-Alanyl-L-Glutamin pro ml umfasst.

14. L-Glutamin zur Verwendung nach einem der Ansprüche 1 bis 14, wobei die Zusammensetzung 1 bis 300 mg, vorzugsweise 5 bis 200 mg, besonders bevorzugt 10 bis 100 mg Glycyl-L-Glutamin pro ml umfasst.

## Revendications

1. L-glutamine destinée à être utilisée pour la préservation, l'amélioration ou la restauration de la fonction rénale ou le ralentissement et/ou l'atténuation d'un déclin de la fonction rénale, la L-glutamine étant fournie dans une composition administrée par voie parentérale, de préférence par voie intraveineuse, la préservation, l'amélioration ou la restauration de la fonction rénale ou le ralentissement et/ou l'atténuation d'un déclin de la fonction rénale se traduisant par le traitement d'une maladie rénale chronique, de préférence une néphropathie chronique progressive, et la préservation, l'amélioration ou la restauration de la fonction rénale ou le ralentissement et/ou l'atténuation d'un déclin de la fonction rénale impliquent le traitement ou la prévention de la glomérulosclérose et/ou de la basophilie tubulaire et/ou de l'atrophie tubulaire et/ou de l'épaississement de la membrane basale tubulaire.

2. L-glutamine destinée à être utilisée selon la revendication 1, le traitement se traduisan par le ralentissement et/ou l'atténuation du développement et/ou l'inversion de la glomérulosclérose et/ou de la basophilie tubulaire et/ou de l'atrophie tubulaire et/ou de l'épaississement de la membrane basale tubulaire.

3. L-glutamine destinée à être utilisée selon l'une quelconque des revendications précédentes chez les patients souffrant d'insuffisance rénale.

4. L-glutamine destinée à être utilisée selon la revendication 5, l'insuffisance rénale se traduisant par une réduction du taux de filtration glomérulaire (TFG).

5. L-glutamine destinée à être utilisée selon la revendication 5 ou 6, l'insuffisance rénale se traduisant par une clairance de la créatinine de 15 à 89 mL par minute.

6. L-glutamine destinée à être utilisée selon l'une quelconque des revendications 5 à 7, l'insuffisance rénale se traduisant par une clairance de la créatinine de 60 à 89 mL par minute.

7. L-glutamine destinée à être utilisée selon l'une quelconque des revendications 5 à 7, l'insuffisance rénale se traduisant par une clairance de la créatinine de 45 à 59 mL par minute.

8. L-glutamine destinée à être utilisée selon l'une quelconque des revendications 5 à 7, l'insuffisance rénale se traduisant par une clairance de la créatinine de 25 à 44 mL par minute.

9. L-glutamine destinée à être utilisée selon l'une quelconque des revendications précédentes chez les patients souffrant de l'un quelconque parmi le diabète, l'ischémie, les infections, l'intoxication, l'hypertension, la septicémie, la glomérulosclérose segmentaire focale, la néphropathie de reflux, la drépanocytose, la maladie auto-immune, la malnutrition, la cachexie, l'inflammation, l'hyperuricémie, et/ou chez les patients admis dans une unité de soins intensifs et/ou chez les patients traumatisés et/ou chez les patients en phase post-chirurgicale.

10. L-glutamine destinée à être utilisée selon l'une quelconque des revendications précédentes, la dose quotidienne de glutamine étant de 50 à 5 000, de préférence de 100 à 4 500 mg par kg de poids corporel.

11. L-glutamine destinée à être utilisée selon l'une quelconque des revendications précédentes, la composition fournissant de 1 à 500 mg, de préférence de 5 à 300 mg, plus préférentiellement de 10 à 150 mg de L-glutamine par mL.

12. L-glutamine destinée à être utilisée selon l'une quelconque des revendications précédentes, la composition comprenant de la L-glutamine en tant qu'acide aminé libre ou sous la forme d'un dipeptide, tripeptide ou tétrapeptide, de préférence sous la forme d'un comprimé, plus préférablement sous la forme de L-alanyl-L-glutamine ou de glycyl-L-glutamine.

13. L-glutamine destinée à être utilisée selon l'une quelconque des revendications précédentes, la composition comprenant de 5 à 550 mg, de préférence de 10 à 400 mg, plus préférentiellement de 20 à 250 mg de L-alanyl-L-glutamine par mL.

14. L-glutamine destinée à être utilisée selon l'une quelconque des revendications 1 à 14, la composition comprenant de 1 à 300 mg, de préférence de 5 à 200 mg, plus préférentiellement de 10 à 100 mg de glycyl-L-glutamine par mL.
